# EUROPEAN PATENT APPLICATION

(11) **EP 1 930 003 A1**
(43) Date of publication of application: **11.06.2008**
(21) Application number: 06769362.2
(22) Date of filing: 06.06.2006
(51) Int. Cl.: A61K 31/27, A61K 31/541, A61K 31/573, A61P 19/02

(54) **PHARMACEUTICAL FORM CONTAINING METHOCARBAMOL, MELOXICAM AND BETAMETHASONE**

(30) Priority: 29.09.2005 MX PA05010505
(71) Applicant: Espinosa Abdala, Leopoldo de Jesús, C.P. 45110 Zapopan, Jalisco , México (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco (MX); ÁLVAREZ OCHOA, Víctor Guillermo, C.P. 45222 Zapopan, Jalisco (MX); SANTOS MURILLO, Josefina, C.P. 44600 Zapopan, Jalisco (MX); FLORES BARBA, Sandra Cecilia, C.P. 45170 Zapopan, Jalisco (MX); ÁLVAREZ ÁLVAREZ, Aracely, C.P. 45235 Tiaquepaque, Jalisco (MX)
(74) Representative: Carvajal y Urquijo, Isabel
(86) International application number: PCT/MX2006/000048
(87) International publication number: WO 2007/037666

(57) **Abstract**

The present invention relates generally to the pharmaceutical industry, and particularly to the pharmaceutical industry dedicated to the preparation of medicaments for the treatment of exacerbations and acute cases of patients suffering from rheumatoid arthritis, ankylosing spondylitis, osteoarthritis, pain and inflammation relief from acute gouty arthritis, acute and subacute bursitis, tendonitis, synovitis.

## Description

### FIELD OF THE INVENTION

The present invention relates generally to the pharmaceutical industry, and particularly to the pharmaceutical industry dedicated to the preparation of medicaments for the treatment of exacerbations and acute cases of patients suffering from rheumatoid arthritis, ankylosing spondylitis, osteoarthritis, pain and inflammation relief from acute gouty arthritis, acute and subacute bursitis, tendonitis, synovitis.

### BACKGROUND OF THE INVENTION

Solid pharmaceutical forms are preparations that contain the active principle(s) and additives, generally disc-shaped, scored or non-scored, and of varying size that are obtained by compressing powders or granules into capsules, patches, pessaries, beads, suppositories, troches or lozenges.

A suspension is a biphasic system consisting in a finely divided solid dispersed in a solid, a liquid or a gas.

An emulsion is a heterogeneous system comprised of two non-miscible liquids, wherein the dispersed phase is comprised of small droplets distributed in the vehicle in which they are immiscible. The active principle(s) can be in the external or the internal phase.

A solution is a liquid, clear and homogeneous preparation obtained by dissolving the active principle(s) and additives in water, and which is employed for external or internal use. In the case of injectable, eye or ear solutions, they should be sterile solutions

A semi-solid is a preparation that up to certain point is a solid having a stiffness and a viscosity in between that of a solid and a liquid.

A syrup is an aqueous solution with a high content of carbohydrates such as: sucrose, sorbitol, dextrose, etc, of viscous consistency in which the active principle(s) and the additives are dissolved.

Methocarbamol has the chemical name 3-(2-methoxyphenoxy)-1,2-propanediol 1-carbamate and it can be represented by formula (I)

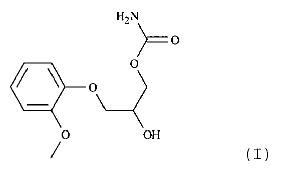

Formula (I) compound is known by its pharmacological activity as musculoskeletal relaxant of central action which although its action in humans has not been well established, it is believed that such action may be due to its sedatives properties in the central nervous system.

Meloxicam is chemically designated as 4-hydroxy-2-methyl-N-(5-methyl-2-thiazolyl)-2H-1,2-benzothiazine-3-carboxamide-1,1-dioxide and it can be represented by the formula (II)

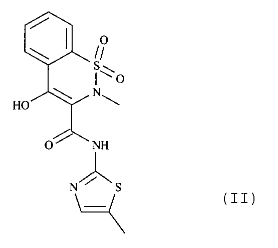

Formula (II) compound is known by its anti-inflammatory, analgesic and anti-pyretic activities which acts by inhibiting prostaglandin synthesis with greater potency in the inflammation site than on kidney and gastric mucosa. Currently is indicated in the treatment of rheumatoid arthritis, osteoarthritis, periarthritis in the scapulohumeral and coxofemoral joints, muscular stress and pain and inflammation in soft tissues and airways exhibiting inflammatory processes.

Bethamethasone has the chemical designation (11β,16β)-9-fluoro-11,17,21-trihydroxy-16-methylpregna-1,4-diene-3,20-dione and it can be represented by formula (III):

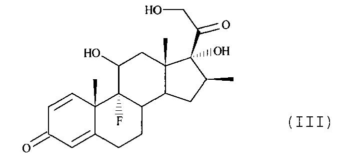

Formula (III) compound is known by its activity as an anti-inflammatory, anti-allergic and anti-rheumatic glucocorticoid. The exact mechanism of action of corticosteroids is unknown. At pharmacological doses, naturally occurring glucocorticoids (cortisone and hydrocortisone) and their synthetic analogues, like bethamethasone, upon administration bind to corticosteroid-binding globulin and in lesser proportion to albumin. The binding affinity helps in the determination of biological half-life of glucocorticoids, which in the case of bethamethasone is from about 72 hours.

### SUMMARY OF THE INVENTION

The present invention provides novel formulations in different pharmaceutical forms comprising: (a) Methocarbamol, (b) Meloxicam, (c) Bethamethasone, (d) one or more anti-adherent agents, (e) one or more disintegrating agents, (f) one or more binding agents, (g) one or more lubricating agents, (h) one or more diluting agents, (i) one or more flavoring and/or perfuming agents, (j) one or more surface active agents, (k) one or more wetting agents, (1) one or more suspending agents, (m) one or more sweetening agents, (n) one or more flocculating agents, (o) one or more preserving agents, (p) one or more antimicrobial agents, (q) one or more buffering agents, (r) one or more solvents and or vehicles, (s) one or more tonicity agents, (t) one or more antioxidants, (u) one or more chelating and/or complexing agents, (v) and any other additive required for the formulation.

The present invention also provides a method for treatment of acute cases and exacerbations of patients suffering from rheumatoid arthritis, ankylosing spondylitis, osteoarthritis, pain and inflammation relief from acute gouty arthritis, acute and subacute bursitis, tendonitis, synovitis
Drug concentrations in the formulation are in an amount from 0.001% to 100.0%

The present invention is characterized in that the combined drugs therein can be found as it anhydrous, monohydrated or dehydrated base or as a physiologically acceptable salt.

### DETAILED DESCRIPTION OF THE INVENTION

To make a pharmaceutical form a series of ingredients can be employed such as: anti-adherents such as: talc, colloidal silicon dioxide, calcium sulfate, calcium chloride, among others, with colloidal silicon dioxide being preferred. The anti-adhering agent should be contained in an amount varying from 0.01% to 10.0% with respect to the formulation total weight.

Disintegrants such as corn starch, modified starches, water soluble cellulose derivatives, sodium crosscarmelose, starch sodium glycolate, binders such as gelatin, natural gums, cellulose derivatives, corn starch, rice starch, polyvynilpirrolidone, povidone, calcium alginate, polyethylene glycols, corn syrup, sucrose, among others, with sodium crosscarmelose being preferred. The disintegrating agent should be contained in an amount varying from 0.25% to 20.0% based on the total weight of the formulation.

Lubricants such as stearic acid, magnesium stearate, talc, among others, with magnesium stearate being preferred. The lubricating agent should be contained in an amount varying from 0.01% to 10.0% with respect to the total weight of the formulation.

Diluents such as microcrystalline cellulose, lactoses, dextrose, sucrose, fructose, phosphates, among others, with sucrose being preferred. The diluting agent should be contained in an amount varying from 0.01% to 90.0% with respect to the formulation total weight.

Surfactants such as poloxamers, sodium lauryl sulfate, sodium docusate, lecithin, polyoxyethylene (20) sorbitan monooleate, among others, with the poloxamers being preferred. The surfactant agent should be contained in an amount varying from 0.01% to 20.0% with respect to the formulation total weight.

The wetting agents can be polar and non-polar, such as ethyl alcohol, water, propylene glycol, polyethylene glycol, glycerol, dimethylacetamide, lecithin, PEG-40 castor oil, butylene glycol, among others, preferably alcohols of 2 carbons such as ethyl alcohol are employed contained in an amount from 1.0% to 50.0% with respect to the total weight of the formulation, excluding the alcohol, and with water being preferred as the polar solvent, contained in an amount varying from 0.01% to 90.0% with respect to the total weight of the formulation.

Flavoring and/or perfuming agents either in powder or liquid form, such as vanilla, cherry, apple, grape, banana, strawberry, peppermint, pineapple, raspberry, chocolate, coconut, peach, lemon, gooseberry, lime, orange, tangerine, among others, synthetic or naturally occurring, with cherry and grape flavors being preferred. The flavoring agent(s) should be contained in an amount from 0.10% to 15% with respect to the total weight of the formulation.

Sweetener(s) such as aspartame, acesulfame K, glutamate monosodium, saccharine, saccharine sodium, among others, with acesulfame K and aspartame being preferred. The sweetener agent should be contained in an amount from 0.00001% to 5.0% with respect to the total weight of the formulation.

Viscosifying agent such as guar gum, acacia gum, sodium carboxymethylcellulose, calcium carboxymethyl cellulose, bentonite, carbomers, carragenine, microcrystalline cellulose and sodium carboxymethyl cellulose dextrines, hydroxypropylmethyl cellulose, hydroxypropyl cellulose, tragacanth gum, xanthan gum, povidone, pectine, methylcellulose, among others, with sodium carboxymethylcellulose being preferred. The viscosifying agent should be in an amount from 0.01% to 20.0% with respect to the total weight of the formulation.
pH buffering system such as phosphates, citrates, carbonates, bicarbonates, acetates, lactates, among others, with phosphates being preferred. The pH buffering system should be contained in an amount from 0.01% to 10% of the formulation total weight.

Flocculating agent such as sodium chloride, potassium chloride, trisodium citrate, among others, with sodium chloride being preferred. The flocculating agent should be in an amount from 0.0001% to 10% of the total weight of the formulation.

Antimicrobials such as parabenes, thymerosal, sodium benzoate, sorbic acid, potassium sorbate, benzyl alcohol and all the antimicrobials that could be used for these pharmaceutical forms. The antimicrobial should be in an amount from 0.0001% to 5% of the formulation total weight.

Solvents and/or vehicles such as ethyl alcohol, corn, sesame, mineral, cottonseed, almond, peanut and soybean oils, glycerin, isopropyl alcohol, polyethylene glycol, propylene glycol, water, sorbitol, ethyl oleate and all solvents and/or vehicles that could be used for these pharmaceutical forms. The solvent and/or vehicle should be in an amount from 0.001% to 99% of the formulation total weight.

Tonicity agents such as dextrose, glycerin, mannitol, sodium and potassium chloride and all the tonicity agents that could be used for these pharmaceutical forms. The tonicity agent should be in an amount from 0.001% to 50% of the formulation total weight.

Antioxidant agents such as ascorbic acid, ascorbyl palmitate, hydroxytoluene butylated, hydroxyanisol butilated, sodium metabisulfite, tocopherols, sodium thiosulfate and all the antioxidant agents that could be used for these pharmaceutical forms. The antioxidant agent should be in an amount from 0.001% to 5.0% of the formulation total weight.

Chelating and/or complexing agents such as disodium edetate, edetic acid, cyclodextrines and all the chelating and/or complexing agents that could be used for these pharmaceutical forms. The chelating and/or complexing agent should be in an amount from 0.001% to 10.0% of the formulation total weight.

Sequestering agents such as cyclodextrines and all the sequestering agents that could be used for these pharmaceutical forms. The sequestering agent should be in an amount from 0.001% to 20.0% of the formulation total weight.

The formulation can be coated using as a coating material the methacrylates, cellulose derivatives, a blend of polymers and polysaccharides for coating by film formation with aqueous or organic solvents, with coloring agents, flavoring agents, sugars and any other ingredient that is used for film forming, coating and dragee making applications, preferably in an sufficient amount to obtain the desired protection of the pharmaceutical form. Optionally, the formulation can also be scored or non-scored. Optionally, the formulation can be filled in hard or soft gelatin capsules.

The formulation, optionally, can contain in addition other components such as stabilizing agents like creatinine or dextrines, adsorbents such as bentonite, magnesium carbonate, powdered cellulose, colorants and/or pigments such as caramel, ferric oxide, red, yellow, black, mixture thereof, and all the colorants and/or pigments that could be used for these pharmaceutical forms.

The formulations can be contained in containers with suitable capacity which can include a blisterpack made of P.V.C. (polyvinyl chloride) film with a thickness from 200 µ to 250 µ, which optionally can have a coating of P.V.D.C. (polyvinylidene chloride) with a weight from 25 g/m² to 120 g/m², and bonded with aluminum foil or in cellopolyal film and also in vials of suitable capacity elaborated from high and/or low density polyethylene, polyethylene terephthalate, polyvinyl chloride, polypropylene, polystyrene, Type I, II, III and IV glass, and any other primary packaging material, with or without color. The cap may be of the following types: tamperproof, threaded, cap-to-cap, child proof, elaborated from high and/or low density polyethylene, polyethylene terephthalate, polyvinyl chloride, polypropylene, polystyrene, with or without color. Dosing auxiliaries, such as dosing spoons, metering cups, pipettes, small syringes, inserts elaborated from materials such as, *inter* alia, high and/or low density polyethylene, polyethylene terephthalate, polyvinyl chloride, polypropylene, polystyrene, with or without color, are also included.

The elaboration is carried out mixing all the components required for the formulation.

Methocarbamol, upon its oral administration, is rapidly absorbed and almost completely in the gastrointestinal tract. Methocarbamol has a serum half-life from 0.9 to 1.8 hours, it is extensively metabolized, presumably in liver, by dealkylation and hydroxylation. The drug and its metabolites are excreted rapidly and almost totally through urine.

Meloxicam exhibits good absorption after being administered orally and 99% of meloxicam binds to plasma proteins. The drug has a good distribution in the body, but particularly it achieves high penetration into synovial fluid, reaching levels that are equivalent to half the concentrations in plasma. Meloxicam is metabolized mainly through oxidation of the methyl group in the thiazolyl molecule. About 50% of the dose is eliminated through urine and the remaining is excreted in feces. The clearance half-life is 20 hours.

Bethamethasone is a glucocorticoid having potent activity, rapidly absorbed in the gastrointestinal tract and distributed to all the body tissues. Bethamethasone binds to plasma proteins, in greater amount to corticosteroid-binding globulin and in a lesser amount to albumin, which means that it has high affinity but low binding ability. It has an extended half-life of 72 hours with an equivalent dose of 0.75 mg; bethamethasone is metabolized in liver and kidney and is excreted through urine.

For treatment of acute cases and exacerbations of patients suffering from rheumatoid arthritis, ankylosing spondylitis, osteoarthritis, pain and inflammation relief from acute gouty arthritis, acute and subacute bursitis, tendonitis, synovitis. The use of 2.25 mg Bethamethasone, from 22.5 to 45 mg Meloxicam and 645 mg Methocarbamol, daily, divided in 3 doses, is recommended.

Therefore, the present invention provides a pharmaceutical formulation, which provides from 50.00 mg to 500.0 mg Methocarbamol, from 1.00 to 30.00 mg Meloxicam and 0.10 mg to 5.00 mg Bethamethasone daily.

### EXAMPLES

The following non-limiting examples will assist to illustrate the present invention:

### EXAMPLE 1

The values are in percent wt/wt

| | | |
|---|---|---|
| 1) | Methocarbamol | 40.0000 |
| 2) | Meloxicam | 0.0500 |
| 3) | Bethamethasone | 0.0050 |
| 4) | Sucrose for compression | 50.0000 |
| 5) | Polyvidone k-30 | 5.0000 |
| 6) | 96% Ethyl alcohol | 20.0000 |
| 7) | Talc | 5.0000 |
| 8) | Purified water | 4.0000 |

The preparation of capsules is carried out as follows: A solution is prepared mixing the solvents, purified water and 96% ethyl alcohol, to this solution Povidone is added and dissolved therein. Next, the active principles Methocarbamol, Meloxicam and Bethamethasone are mixed and then wetted with the solution that contains Povidone. The resulting mixture is dried, screened and thereafter sucrose for compression and talc are added. Proceed to encapsulation.

### EXAMPLE 2

The values are in percent wt/vol

| | | |
|---|---|---|
| 1) | Methocarbamol | 30.000 |
| 2) | Meloxicam | 0.500 |
| 3) | Bethamethasone | 0.050 |
| 4) | Sodium carboxymethyl cellulose | 10.000 |
| 5) | Colloidal silicon dioxide | 1.000 |
| 6) | 96% ethyl alcohol | 2.000 |
| 7) | Dextrose | 70.000 |
| 8) | Flavor | 0.500 |
| 9) | Color | 0.010 |
| 10) | Propylene glycol | 20.000 |
| 11) | Sodium benzoate | 0.010 |
| 12) | Purified water, q.s. | 100.000 |

The elaboration of the suspension is carried out as follows: carboximethyl cellulose, dextrose, sodium benzoate, coloring agent and flavoring agent are dissolved in a portion of water. Then, 96% ethyl alcohol, propylene glycol and colloidal silicon dioxide are added, this is stirred to homogenize the suspension, then Bethamethasone, Meloxicam and Methocarbamol are added and the mixture is stirred until an homogeneous suspension is obtained. Finally water is added to the desired volume.

### EXAMPLE 3

The values are in percent wt/wt

| | | |
|---|---|---|
| 1) | Methocarbamol | 0.20 |
| 2) | Meloxicam | 0.05 |
| 3) | Bethamethasone | 0.50 |
| 4) | Carbomer | 10.00 |
| 5) | Sodium hydroxide | 5.00 |
| 6) | 96% ethyl alcohol | 4.00 |
| 7) | Propyl parabene | 2.00 |
| 8) | Methyl parabene | 0.01 |
| 12) | Purified water, q.s. | 80.00 |

The elaboration of in an amount of purified water the carbomer is dispersed, once it is dispersed, in a separate vessel the sodium hydroxide is dissolved and then this is added slowly and with continuous stirring to the carbomer dispersion. The 96% ethyl alcohol is added to another amount of purified water and the propyl parabene, methyl parabene, bethamethasone, meloxicam and methocarbamol are dissolved in this solution. Add the active-containing solution and this is added to the matrix and stirred until homogenization.

The invention has been described sufficiently as to allow that a person of ordinary skill in the art could realize and obtain the results mentioned in the present invention. However, any person of ordinary skill in the art to which the present invention pertains could be able to make modifications not disclosed in the present application, nonetheless, if for the application of these modifications in a given structure or in the manufacturing process thereof, the subject matter claimed in the following claims is required, said structures should be embraced within the scope of the invention.

## Claims

1. A pharmaceutical form which contains Methocarbamol, Meloxicam, Bethamethasone, one or more anti-adherent agents, one or more disintegrating agents, one or more binding agents, one or more lubricating agents, one or more diluting agents, one or more flavoring and/or perfuming agents, one or more surface active agents, one or more wetting agents, one or more suspending agents, one or more sweetening agents, one or more flocculating agents, one or more preserving agents, one or more antimicrobial agents, one or more pH buffering systems, and one or more solvents and/or vehicles, one or more tonicity agents, one or more antioxidants, one or more chelating agents, and any other additive required for the formulation.

2. A formulation as claimed in claim 1, wherein the concentrations of the active principles in the formulation are in an amount from 0.001% to 100.0%.

3. A formulation as claimed in claim 2, wherein the combined active principles therein can be found as it anhydrous, monohydrated or dehydrated base or as a physiologically acceptable salt, with Methocarbamol, Meloxicam and Bethamethasone being preferred.

4. A formulation as claimed in claim 3, wherein it contains one or more of the following ingredients: anti-adherents such as: talc, colloidal silicon dioxide, calcium sulfate, calcium chloride, among others. Disintegrants such as corn starch, modified starches, water soluble cellulose derivatives, sodium crosscarmelose, starch sodium glycolate, binders such as gelatin, natural gums, cellulose derivatives, corn starch, rice starch, polyvynilpirrolidone, povidone, calcium alginate, polyethylene glycols, corn syrup, sucrose. Lubricants such as stearic acid, magnesium stearate, talc, among others. Diluents such as microcrystalline cellulose, lactoses, compressible sugar, dextrose, sucrose, fructose, among others. Surfactants such as poloxamers, sodium lauryl sulfate, sodium docusate, lecithin. The wetting agents can be polar and non-polar, such as ethyl alcohol, water, propylene glycol, polyethylene glycol, glycerol, dimethylacetamide, lecithin, PEG-40 castor oil, butylene glycol, among others. Flavoring and/or perfuming agents either in powder or liquid form, such as vanilla, cherry, apple, banana, strawberry, peppermint, pineapple, raspberry, chocolate, coconut, peach, lemon, gooseberry, lime, orange, tangerine, among others, synthetic or naturally occurring. Sweetener(s) such as aspartame, acesulfame K, monosodium glutamate, saccharine, sodium saccharine, among others. Viscosifying agent such as guar gum, acacia gum, sodium carboxymethylcellulose, calcium carboxymethyl cellulose, bentonite, carbomers, carragenine, microcrystalline cellulose and sodium carboxymethyl cellulose dextrines, hydroxypropylmethyl cellulose, hydroxypropyl cellulose, tragacanth gum, xanthan gum, povidone, pectine, methylcellulose, among others. pH buffering system such as phosphates, citrates, carbonates, bicarbonates, acetates, lactates, among others. Flocculating agent such as sodium chloride, potassium chloride, trisodium citrate, among others. Antioxidant agents such as ascorbic acid, ascorbyl palmitate, hydroxytoluene butylated, hydroxyanisol butilated, sodium metabisulfite, tocopherols, sodium thiosulfate among others. Tonicity agents such as dextrose, glycerin, mannitol, sodium and potassium chloride among others. Solvents and/or vehicles such as ethyl alcohol, corn, sesame, mineral, cottonseed, almond, peanut and soybean oils, glycerin, isopropyl alcohol, polyethylene glycol, propylene glycol, water, sorbitol, ethyl oleate among others. Chelating and/or complexing agents such as disodium edetate (E.D.T.A), ascorbic acid, Butyl hydroxytoluene (BHT), tocopherols, sodium bisulfite, sodium metabisulfite among others. Antimicrobials such as parabenes, thymerosal, sodium benzoate, sorbic acid, potassium sorbate, benzyl alcohol and all the antimicrobials that could be used for these pharmaceutical forms.

5. A formulation as claimed in all the preceding claims, wherein said formulation can be a dragee, tablet, coated tablet, capsule, suspension, syrup, emulsion, troches or lozenges, solution, with capsules being preferred.

6. The formulation that might contain a coating agent, employing as a coating material the methacrylates, cellulose derivatives, a blend of polymers and polysaccharides for coating by film formation with aqueous or organic solvents, which also has coloring agents, flavoring agents, sugars and any other ingredient that is used for film forming, coating and dragee making applications.

7. A formulation that might be encapsulated in hard or soft gelatin capsules. The formulation that might contain in addition other components such as stabilizing agents like creatinine or dextrines among others. Furthermore, adsorbents such as bentonite, magnesium carbonate, powdered cellulose,among other may also be included.

8. A formulation as claimed in claim 7, wherein said formulation is contained in containers with suitable capacity which can include a blister pack made of P.V.C. (polyvinyl chloride) film with a thickness from 200 µ to 250 µ, which optionally can have a coating of P.V.D.C. (polyvinylidene chloride) with a weight from 25 g/m² to 120 g/m², and bonded with aluminum foil or in cellopolyal film and also in bottles of suitable capacity elaborated from high and/or low density polyethylene, polyethylene terephthalate, polyvinyl chloride, polypropylene, polystyrene, Type I, II, III and IV glass, and any other primary packaging material, with or without color. The cap may be of the following types: tamperproof, threaded, cap-to-cap, child proof, elaborated from high and/or low density polyethylene, polyethylene terephthalate, polyvinyl chloride, polypropylene, polystyrene, with or without color. Dosing auxiliaries, such as dosing spoons, metering cups, pipettes, small syringes, inserts elaborated from materials such as, *inter alia,* high and/or low density polyethylene, polyethylene terephthalate, polyvinyl chloride, polypropylene, polystyrene, among others, with or without color, are also included.

9. A method for treatment of acute cases and exacerbations of patients suffering from rheumatoid arthritis, ankylosing spondylitis, osteoarthritis, pain and inflammation relief from acute gouty arthritis, acute and subacute bursitis, tendonitis, synovitis, by administering from 50.00 mg to 500.00 mg of Methocarbamol, from 1.00 mg to 30.00 mg of Meloxicam and from 0.10 mg to 5.00 mg of Bethamethasone daily.

10. A solid pharmaceutical formulation which provides from 50.00 mg to 500.0 mg Methocarbamol, from 1.00 to 30.00 mg Meloxicam and from 0.10 mg to 5.00 mg Bethamethasone daily.
